# EUROPEAN PATENT APPLICATION

(11) **EP 4 491 200 A1**
(43) Date of publication of application: **15.01.2025**
(21) Application number: 24187687.9
(22) Date of filing: 10.07.2024
(51) Int. Cl.: A61L 9/013, A61L 9/14, B01D 53/34, B01D 53/44, B01D 53/48, B01D 53/58, B01D 53/76

(54) **DEODORANT FRAGRANCE COMPOSITION**

(30) Priority: 13.07.2023 JP 2023115283
(71) Applicant: Takasago International Corporation, Tokyo 144-8721 (JP)
(72) Inventor: KUWAHARA, Yukari, Hiratsuka-shi, Kanagawa, 25400773 (JP)
(74) Representative: Hoffmann Eitle

(57) **Abstract**

[Problem]

To identify an unpleasant odor that is a feces and urine odor of dogs and cats and to provide a fragrance composition, a pet product, and the like capable of reducing the unpleasant odor.

[Solution to Problem]

As a result of studies on a fragrance capable of strongly masking, for a long time, an odor generated by a compound that causes an unpleasant odor, the present inventors have found that a specific fragrance has an extremely high masking effect. The present invention can provide a fragrance composition having an effect of deodorizing (masking) a feces and urine odor of dogs and cats, and a deodorant and a pet product containing the fragrance composition.

## Description

### TECHNICAL FIELD

The present invention relates to a fragrance or a fragrance composition for masking unpleasant smells generated from feces and urine of dogs and cats.

### BACKGROUND ART

When a pet, in particular, a dog or a cat is fed, a problem of feces and urine odors may occur. Although the pet feces and urine odor has been researched so far, ammonia and fatty acids have been at the top of the list, and 3-mercapto-3-methylbutanol (hereinafter referred to as 3MMB) has been often cited especially for a cat urine odor. However, there are case that 3MMB may not be detected in a sample, and thus, it is considered that malodorous components other than 3MMB contribute to the discomfort of owners of the pets. However, the component has not been identified, and no effective deodorization method or masking method has been found.

On the other hand, a fragrance composition for deodorizing a pet odor has been known. The fragrance composition has an effect of deodorizing a urine odor of pets or animals and an excrement odor of cats. For example, Patent Literature 1 and Patent Literature 2 disclose a deodorant fragrance composition containing a specific fragrance component.

### CITATION LIST

### PATENT LITERATURE

Patent Literature 1: US2009/0238787A1
Patent Literature 2: JP2016131495A

### NON-PATENT LITERATURE

Non-Patent Literature 1: Chemistry and Biology, 13, p1071-1079 (2006)

### SUMMARY OF INVENTION

### TECHNICAL PROBLEM

An object of the present invention is to identify an unpleasant odor that is a feces and urine odor of dogs and cats, and to provide a fragrance composition, a pet product, and the like capable of reducing the unpleasant odor.

### SOLUTION TO PROBLEM

In order to search for a masking agent effective for an unpleasant odor that is a feces and urine odor of dogs and cats (in particular, dogs and cats at the age of 7 years or more), the present inventors have identified the unpleasant odor and have identified a substance causing smells of the unpleasant odor.

Subsequently, as a result of studies on a fragrance capable of strongly masking, for a long time, an odor generated by a compound that causes an unpleasant odor, the present inventors have found that a specific fragrance has an extremely high masking effect, and have completed the present invention.

That is, the present invention relates to the following contents.
[1] A deodorant fragrance composition for a feces and urine odor of dogs and cats, the deodorant fragrance composition containing one or more selected from the following component A and one or more selected from the following component B.
   Component A: 8-mercaptomenthone, 1-(5,6,7,8-tetrahydro-2-naphthalenyl)ethanone, 2,4,6-trimethyl-4-phenyl-1,3-dioxane, and ethyleneblassylate
   Component B: mint oil, benzyl acetate, citronellol, (2-tert-butylcyclohexyl)acetate, (4-tert-butylcyclohexyl)acetate, allylamylglycolate, linalool, α-Ionone, and allylheptanoate
[2] The deodorant fragrance composition for a urine odor of a dog according to [1], in which the component A is 1-(5,6,7,8-tetrahydro-2-naphthalenyl)ethanone, and the components B are citronellol and allylheptanoate.
[3] The deodorant fragrance composition for a feces and urine odor of a cat according to [1], in which the components A are 8-mercaptomenthone and 2,4,6-trimethyl-4-phenyl-1,3-dioxane, and the components B are mint oil and allylamylglycolate.
[4] A method for masking a feces and urine odor of dogs and cats, the method including:
   using the deodorant fragrance composition according to any one of [1] to [3].
[5] A pet product containing:
   the deodorant fragrance composition according to any one of [1] to [3].
[6] Use of a fragrance composition for deodorizing a feces and urine odor of dogs and cats, the fragrance composition containing one or more selected from the following component A and one or more selected from the following component B.
   Component A: 8-mercaptomenthone, 1-(5,6,7,8-tetrahydro-2-naphthalenyl)ethanone, 2,4,6-trimethyl-4-phenyl-1,3-dioxane, and ethyleneblassylate
   Component B: mint oil, benzyl acetate, citronellol, (2-tert-butylcyclohexyl)acetate, (4-tert-butylcyclohexyl)acetate, allylamylglycolate, linalool, α-Ionone, and allylheptanoate
[7] The use of a fragrance composition for deodorizing a urine odor of a dog according to [6], in which the component A is 1-(5,6,7,8-tetrahydro-2-naphthalenyl)ethanone, and the components B are citronellol and allylheptanoate.
[8] The use of a fragrance composition for deodorizing a feces and urine odor of a cat according to [6], in which the components A are 8-mercaptomenthone and 2,4,6-trimethyl-4-phenyl-1,3-dioxane, and the components B are mint oil and allylamylglycolate.

### ADVANTAGEOUS EFFECTS OF INVENTION

The present invention can provide a fragrance composition having an effect of deodorizing (masking) a feces and urine odor of dogs and cats, and a deodorant and a pet product containing the fragrance composition.

### DESCRIPTION OF EMBODIMENTS

In the present disclosure, examples of a compound contained in the feces and urine odor of dogs and cats, in particular, a compound which is increased when they become 7 years old or more include, for example, sulfides, lactones, pyrazines, and anthranilic acids. Examples of the sulfides include dimethylsulfide. Examples of the lactones include nonan-1,4-olide. Examples of the pyrazines include trimethylpyradine and 2,5-dimethylpyradine. Examples of the anthranilic acids include methylanthranilate.

The fragrance in the deodorant fragrance composition according to the present invention is preferably one or more selected from the following components A and B.
Component A: 8-mercaptomenthone, 1-(5,6,7,8-tetrahydro-2-naphthalenyl)ethanone, 2,4,6-trimethyl-4-phenyl-1,3-dioxane, and ethyleneblassylate
Component B: mint oil, benzyl acetate, citronellol, (2-tert-butylcyclohexyl)acetate, (4-tert-butylcyclohexyl)acetate, allylamylglycolate, linalool, α-Ionone, and allylheptanoate

The fragrances of the component A and the component B described above are preferably contained in an amount of 0.001 mass% to 30 mass%, more preferably contained in an amount of 0.01 mass% to 15 mass%, further preferably contained in an amount of 0.1 mass% to 10 mass% in the fragrance composition according to the present invention.

Among the fragrances described above, 1-(5,6,7,8-tetrahydro-2-naphthalenyl)ethanone, citronellol, and allylheptanoate are preferred as fragrance components for deodorizing a urine odor of dogs.

Among the fragrances described above, 8-mercaptomenthone, 2,4,6-trimethyl-4-phenyl-1,3-dioxane, mint oil, and allylamylglycolate are preferred as fragrance components for deodorizing a feces and urine odor of cats.

When the fragrance composition according to the present invention is blended into a pet product such as a pet sheet or cat litter, the fragrance composition is preferably contained in an amount of about 0.01 mass% to 50 mass%, more preferably contained in an amount of about 0.05 mass % to 30 mass%, and further preferably contained in an amount of 0.1 mass % to 15 mass% depending on the type of the fragrance compound.

In the present invention, masking is referred to as a combination of functions such as shielding, covering, deodorization, and removal of an unpleasant odor using a fragrance compound or a fragrance composition.

The fragrance composition according to the present invention may contain, in addition to the deodorant fragrance described above, a scenting fragrance described below, and may further contain, as necessary, a basic agent, an additive, and the like that are generally used.

### (1) Scenting Fragrance

A blending amount of the scenting fragrance is within a range where a masking effect of the fragrance composition according to the present invention is not affected. The scenting fragrance is not particularly limited, and examples thereof include the following fragrance compounds.

Preferred examples of the fragrance compounds include: esters formed by linear, branched, or cyclic saturated or unsaturated aliphatic carboxylic acids having 1 to 20 carbon atoms or aromatic carboxylic acids having 6 to 10 carbon atoms and linear, branched, or cyclic alcohols having 1 to 10 carbon atoms; linear, branched, or cyclic saturated or unsaturated aliphatic alcohols having 5 to 20 carbon atoms; aromatic alcohols having 6 to 10 carbon atoms; linear, branched, or cyclic saturated or unsaturated aliphatic aldehydes having 6 to 20 carbon atoms; aromatic aldehydes having 6 to 20 carbon atoms; linear, branched, or cyclic saturated or unsaturated aliphatic ketones having 10 to 20 carbon atoms; and aromatic ketones having 6 to 15 carbon atoms.

Examples of preferred fragrance compounds also include: (di)acetals formed by linear, branched, or cyclic saturated or unsaturated aliphatic aldehydes having 2 to 15 carbon atoms and linear, branched, or cyclic saturated or unsaturated aliphatic alcohols (or diols) having 1 to 10 carbon atoms; and (di)acetals formed by linear, branched, or cyclic saturated or unsaturated aliphatic ketones having 2 to 15 carbon atoms and linear, branched, or cyclic saturated or unsaturated aliphatic alcohols (or diols) having 1 to 10 carbon atoms.

Examples of preferred compounds include: phenols such as eugenol and thymol; ethers such as anethol, 1,8-cineol, linalool oxide, limonen oxide, rose oxide, rose furan, and theaspiran; γ-lactones or δ-lactones having 6 to 20 carbon atoms; furans such as 3-(2-furyl)-2-methyl-2-propenal, 2,5-dimethyl-4-hydroxy-3(2H)-furanone, and sotolon; hydrocarbons such as β-caryophyllene, terpinene, limonene, and α-pinen or β-pinen; and linear, branched, or cyclic saturated or unsaturated aliphatic carboxylic acids having 5 to 20 cyclic carbon atoms.

Natural fragrances such as orange, lemon, lavender, rose, sandalwood, jasmine, eucalyptus, peppermint, spearmint, rosemary, lavandin, and vanilla may also be used.

One of these fragrances may be used alone, or two or more thereof may be used in combination.

### (2) Other Components

A masking fragrance composition according to the present invention may further contain, for example, optional components generally used for deodorizing feces and urine odors of pets (dogs and cats). One of the optional components may be used alone, or two or more thereof may be used in combination. Examples of the optional components include antibacterial agents, other deodorants (deodorants other than the active ingredients of the deodorants according to the present invention), bactericides, preservatives, fungicides, and organic acids and/or salts thereof. Among them, it is preferable to contain an antibacterial agent and other deodorants.

The pet products into which the deodorant fragrance composition according to the present invention is blended may be used as a space deodorant (an air freshener, an air detergent, or the like), a clothing deodorant, a deodorant for excrement, or the like, depending on a target and purpose for spraying. The deodorant may be used in the form of solution, spray, cream, paste, gel, powder, granule, or the like, and is preferably a spray. When the deodorant according to the present invention is a spray agent, the spray agent may be either an aerosol type or a pump type. The spray agent can be used by being sprayed into pet excrement, a sheet that has absorbed excrement, cat litter, clothing, a space (room or the like), or the like.

The deodorant fragrance composition according to the present invention can effectively mask an unpleasant odor that is a feces and urine odor of pets (dogs and cats) by being blended into various pet products. The blending amount in the pet products is preferably 0.01 mass% to 50 mass%, more preferably contained in an amount of about 0.05 mass % to 30 mass%, and further preferably contained in an amount of 0.1 mass % to 15 mass%.

### [Examples]

Hereinafter, the present invention will be described in more detail with reference to Examples, but the present invention is not limited thereto.

### (Identification of Key Substances Causing Unpleasant Odor)

The main components of the feces and urine odor of dogs and cats were identified as follows. First, 40 actual dog feces samples, 10 actual dog urine samples, 10 actual cat feces samples, and 10 actual cat urine samples were obtained, and then the samples were analyzed using SPME-GC/MS.

As a result of the analysis, sulfides, lactones, pyrazines, and anthranilic acids were detected. Among them, in particular, components whose detected amount had a significant difference between dogs and cats at the age of 7 years or less and dogs and cats at the age of 7 years or more and which sensually contributed to the feces and urine odor were combined to prepare compositions shown in Tables 1 to 3. The compositions generate the feces and urine odor. In the tables, DPG represents dipropylene glycol.

**Table 1**

| Composition for generating feces odor of cats | Blending ratio (mass%) |
|---|---|
| Caproic acid | 30.0 |
| Isovaleric acid | 30.0 |
| Butanoic acid | 0.3 |
| Indole | 1.5 |
| Methyl anthranilate | 0.5 |
| Triethyl citrate | Remaining amount |

**Table 2**

| Composition for generating urine odor of cats | Blending ratio (mass%) |
|---|---|
| Anmmonia 30% aqueous solution | 60.0 |
| Isoamylalcohol 1% dipropylene glycol solution | 15.0 |
| p-Cresol 0.1% dipropylene glycol solution | 5.0 |
| Trimethlpyrazine 1% dipropylene glycol solution | 0.2 |
| 2,5-Dimethylpyrazine 1% dipropylene glycol solution | 0.2 |
| Triethyl citrate | Remaining amount |

**Table 3**

| Composition for generating urine odor of dog | Blending ratio (mass%) |
|---|---|
| Anmmonia 30% aqueous solution | 60.0 |
| Isoamylalcohol 1% dipropylene glycol solution | 15.0 |
| p-Cresol 0.1% dipropylene glycol solution | 5.0 |
| Nonan-1,4-olide | 1.0 |
| Dimethylsulfide 0.1% dipropylene glycol solution | 0.2 |

### [Examples 1 to 13] (Evaluation and Selection of Masking Fragrance)

The tests for evaluating a masking effect and a harmonizing effect on the feces and urine odor were performed by the following procedure. With 10 µL of each composition shown in Tables 1 to 3 was blended 1 µL of a masking fragrance component, and a cotton ball was impregnated with the mixture. Next, the cotton ball was charged into a plastic bottle, and the plastic bottle was sealed with a lid. After 30 minutes, the lid of the plastic bottle was opened, and the masking effect and the harmonization effect on the feces and urine odor were separately subjected to sensory evaluation. The evaluation values of the masking effect and the harmonization effect are average values of numerical values obtained by sensory evaluation performed by three specialized panelists based on the following evaluation criteria.

### Masking Effect

3: well masked.
2: masked.
1: neither.
0: not mask at all.

### Harmonizing

1: well harmonizing.
0: neither (the same as blank).
-1: not harmonizing.

In the present invention, a compound having a score related to the masking effect of 1.5 or more and a score related to the harmonization effect of 0.5 or more was used as a component excellent in reducing the feces and urine odor.

**Table 4**

| | | For urine of dog | | For urine of cat | | For feces of cat | |
|---|---|---|---|---|---|---|---|
| Examples | Compound name | Masking | Harmonization | Masking | Harmonization | Masking | Harmonization |
| Example 1 | 8-Mercaptomenthone | 2.3 | 0.7 | 3.0 | 1.0 | 3.0 | 1.0 |
| Example 2 | 1-(5,6,7,8-Tetrahydro-2-naphthalenyl)ethanone | 2.7 | 0.7 | 2.8 | 0.8 | 2.8 | 0.8 |
| Example 3 | 2,4,6-Trimethyl-4-phenyl-1,3-dioxane | 2.7 | 0.7 | 2.8 | 0.8 | 3.0 | 1.0 |
| Example 4 | Ethyleneblassylate | 2.0 | 0.5 | 2.0 | 1.0 | 2.2 | 0.5 |
| Example 5 | Mint oil | 2.7 | 0.7 | 3.0 | 1.0 | 3.0 | 1.0 |
| Example 6 | Benzyl acetate | 2.1 | 0.5 | 2.0 | 0.5 | 2.2 | 0.5 |
| Example 7 | Citronellol | 2.9 | 0.7 | 2.7 | 0.6 | 2.7 | 0.6 |
| Example 8 | (2-tert-Butylcyclohexyl)acetate | 2.5 | 0.8 | 2.4 | 0.7 | 2.5 | 0.7 |
| Example 9 | (4-tert-Butylcyclohexyljacetate | 2.6 | 0.9 | 2.5 | 0.7 | 2.8 | 0.7 |
| Example 10 | Allylamylglycolate | 2.7 | 0.7 | 3.0 | 1.0 | 3.0 | 1.0 |
| Example 11 | Linalool | 2.5 | 0.5 | 2.4 | 0.5 | 2.4 | 0.5 |
| Example 12 | α-Ionone | 2.5 | 0.8 | 2.4 | 0.7 | 2.5 | 0.7 |
| Example 13 | Allylheptanoate | 3.0 | 0.9 | 2.8 | 0.9 | 2.8 | 0.9 |

### [Examples 14 to 16] (Use of Deodorant Fragrance Composition in Pet Toilet Sheets)

Next, evaluation was performed in the case where the deodorant fragrance composition was blended into a pet toilet sheet. A fruit scents deodorant fragrance composition, a floral scents deodorant fragrance composition, and a herbal scents deodorant fragrance composition were prepared respectively in accordance with formulations shown in Tables 5a to 5c. A floral fragrance containing no deodorant fragrance composition was prepared as a comparative example in accordance with a formulation shown in Table 6.

### [Evaluation Method]

A pet toilet sheet (double-sided absorbent pet sheet (wide), manufactured by CAINZ CORPORATION) was scented with 30 µl of each deodorant fragrance composition shown in Tables 5a to 5c and a fragrance as the comparative example shown in Table 6, and 100 µl of each model malodor substance shown in Tables 1 to 3 was added dropwise from above.

Three specialized panelists checked the samples prepared in the above steps, and determined the masking effect and the harmonization effect of the samples on an unpleasant odor that is a feces and urine odor according to the following criteria.

### Masking Effect

3: well masked.
2: masked.
1: neither.
0: not mask at all.

### Harmonizing

1: well harmonizing.
0: neither (the same as blank).
-1: not harmonizing.

**Table 5a**

| Fruit scents | Blending amount (%) |
|---|---|
| 8-MercaptoMenthone 1% Dipropylene glycol solution | 1 |
| (2-tert-Butylcyclohexyl)acetate | 20 |
| (4-tert-Butylcyclohexyl)acetate | 10 |
| Allylamylglycolate | 5 |
| Linalool | 24 |
| α-Ionone | 30 |
| Allylheptanoate | 10 |

**Table 5b**

| Floral scents | Blending amount (%) |
|---|---|
| Ethyleneblassylate | 30 |
| Citronellol | 30 |
| Linalool | 20 |
| α-Ionone | 20 |

**Table 5c**

| Herbal scents | Blending amount (%) |
|---|---|
| 1-(5,6,7,8-Tetrahydro-2-naphthalenyl)ethanone | 2 |
| 2,4,6-Trimethyl-4-phenyl-1,3-dioxane | 5 |
| Mint oil | 10 |
| Benzyl acetate | 20 |
| Allylamylglycolate | 20 |
| Linalool | 13 |
| Allylheptanoate | 30 |

**Table 6**

| Floral scents | Blending amount (%) |
|---|---|
| Methyl dihydrojasmonate | 40 |
| Terpineol | 20 |
| Phenylethyl alcohol | 30 |
| Heliotropine | 10 |

The evaluation results are shown in Table 7.

**Table 7**

| | Example 14 | Example 15 | Example 16 | Comparative Example 1 |
|---|---|---|---|---|
| | Blending amount | Blending amount | Blending amount | Blending amount |
| Fruit scents | 30 µl | | | |
| Floral scents | | 30 µl | | |
| Herbal scents | | | 30 µl | |
| Floral scents as Comparative Example | | | | 30 µl |
| Pet sheets | One | One | One | One |
| [Evaluation 1] masking/harmonization for urine of dog | 2.6/0.7 | 2.5/0.6 | 2.9/0.8 | 1.2/0.4 |
| [Evaluation 2] masking/harmonization for urine of cat | 2.5/0.6 | 2.7/0.7 | 2.8/0.8 | 0.8/0.4 |
| [Evaluation 3] masking/harmonization for feces of cat | 2.6/0.6 | 2.7/0.6 | 2.6/0.7 | 0.7/0.0 |

### [Examples 17 to 19] (Use of Deodorant Fragrance Composition in Cat Litter)

As shown in Table 8, cat litter containing the deodorant fragrance composition according to the present invention was prepared. An evaluation method was performed as follows. Cat litter (Pet's One hardening cat litter: CAINZ CORPORATION) was scented with 100 µl of each of the deodorant fragrance compositions shown in Tables 5a to 5c and the fragrance as a comparative example shown in Table 8, and 100 µl of each model malodor substance shown in Tables 1 to 3 was added dropwise from above.

Three specialized panelists checked the samples prepared in the above steps, and determined the masking effect and the harmonizing effect of the samples on an unpleasant odor that is a feces and urine odor.

**Table 8**

| | Example 14 | Example 15 | Example 16 | Comparative Example 2 |
|---|---|---|---|---|
| | Blending amount | Blending amount | Blending amount | Blending amount |
| Fruit scents | 100 µl | | | |
| Floral scents | | 100 µl | | |
| Herbal scents | | | 100 µl | |
| Floral scents as Comparative Example | | | | 100 µl |
| Cat litter (mineral) | 100 g | 100 g | 100 g | 100 g |
| [Evaluation 1] masking/harmonization for urine of dog | 2.8/0.8 | 2.5/0.6 | 2.8/0.8 | 0.8/-0.3 |
| [Evaluation 2] masking/harmonization for urine of cat | 2.7/0.7 | 2.8/0.7 | 2.6/0.8 | 0.5/-0.1 |
| [Evaluation 3] masking/harmonization for feces of cat | 2.7/0.7 | 2.7/0.6 | 2.6/0.7 | 0.3/0.0 |

### [Examples 17 to 19] (Use of Deodorant Fragrance Composition in Cat Litter)

As shown in Table 9, cat litter containing the deodorant fragrance composition according to the present invention was prepared. An evaluation method was performed as follows. Cat litter (Pet's One paper cat litter: CAINZ CORPORATION) was scented with 100 µl of each of the deodorant fragrance compositions shown in Tables 5a to 5c and the fragrance as the comparative example shown in Table 6, and 100 µl of each model malodor substance shown in Tables 1 to 3 was added dropwise from above.

Three specialized panelists checked the samples prepared in the above steps, and determined the masking effect and the harmonizing effect of the samples on an unpleasant odor that is a feces and urine odor.

**Table 9**

| | Example 17 | Example 18 | Example 19 | Comparative Example 3 |
|---|---|---|---|---|
| | Blending amount | Blending amount | Blending amount | Blending amount |
| Fruit scents | 100 µl | | | |
| Floral scents | | 100 µl | | |
| Herbal scents | | | 100 µl | |
| Floral scents as Comparative Example | | | | 100 µl |
| Cat litter (paper) | 100 g | 100 g | 100 g | 100 g |
| [Evaluation 1] masking/harmonization for urine of dog | 2.8/0.8 | 2.5/0.6 | 2.8/0.8 | 0.4/-0.4 |
| [Evaluation 2] masking/harmonization for urine of cat | 2.7/0.7 | 2.8/0.7 | 2.6/0.8 | 0.5/-0.2 |
| [Evaluation 3] masking/harmonization for feces of cat | 2.7/0.7 | 2.7/0.6 | 2.6/0.7 | 0.3/-0.1 |

### [Examples 20 to 22] (Use of Deodorant Fragrance Composition in Pet Shampoo)

As shown in Table 10, a pet shampoo containing the deodorant fragrance composition according to the present invention was prepared. An evaluation method was performed as follows. A shampoo shown in Table 10 was added dropwise onto filter paper, and 10 µl of each model malodor substance shown in Tables 1 to 3 was added dropwise from above.

Three specialized panelists checked the samples prepared in the above steps, and determined the masking effect and the harmonizing effect of the samples on an unpleasant odor that is a feces and urine odor.

**Table 10**

| | Example 20 | Example 21 | Example 22 | Comparative Example 4 |
|---|---|---|---|---|
| Compound name | Blending amount | Blending amount | Blending amount | Blending amount |
| Fruit scents | 0.5 | | | |
| Floral scents | | 0.5 | | |
| Herbal scents | | | 0.5 | |
| Floral scents as Comparative Example | | | | 0.5 |
| Sodium polyoxyethylene lauryl ether sulfate | 28.0 | 28.0 | 28.0 | 28.0 |
| Coconut oil fatty acid amidopropyl betaine solution | 8.0 | 8.0 | 8.0 | 8.0 |
| Methylparaben | 2.0 | 2.0 | 2.0 | 2.0 |
| Phenoxyethanol | 0.3 | 0.3 | 0.3 | 0.3 |
| Sodium lauryl sulfate | 28.0 | 28.0 | 28.0 | 28.0 |
| NIKKOL AM-3130N | 8.0 | 8.0 | 8.0 | 8.0 |
| Purified water | Remaining amount | Remaining amount | Remaining amount | Remaining amount |
| [Evaluation 1] masking/harmonization for urine of dog | 2.8/0.9 | 2.6/0.7 | 2.8/0.8 | 0.5/0.3 |
| [Evaluation 2] masking/harmonization for urine of cat | 2.6/0.8 | 2.8/0.7 | 2.7/0.8 | 0.5/0.1 |
| [Evaluation 3] masking/harmonization for feces of cat | 2.6/0.7 | 2.8/0.7 | 2.6/0.8 | 0.3/0.3 |

### [Examples 23 to 25] (Use of Deodorant Fragrance Composition in Pet Spray)

As shown in Table 11, a pet spray containing the deodorant fragrance composition according to the present invention was prepared. A filter paper to which 10 µl of each model malodor substance shown in Tables 1 to 3 was added dropwise was placed in a disposable beaker, and a spray shown in Table 11 was sprayed from above.

Three specialized panelists checked the samples prepared in the above steps, and determined the masking effect and the harmonizing effect of the samples on an unpleasant odor that is a feces and urine odor.

**Table 11**

| | Example 23 | Example 24 | Example 25 | Comparative Example 4 |
|---|---|---|---|---|
| Compound name | Blending amount | Blending amount | Blending amount | Blending amount |
| Fruit scents | 0.2 | | | |
| Floral scents | | 0.2 | | |
| Herbal scents | | | 0.2 | |
| Floral scents as Comparative Example | | | | 0.2 |
| Ethanol | 10.0 | 10.0 | 10.0 | 10.0 |
| Methyl-β-cyclodextrin | 0.5 | 0.5 | 0.5 | 0.5 |
| Purified water | Remaining amount | Remaining amount | Remaining amount | Remaining amount |
| [Evaluation 1] masking/harmonization for urine of dog | 2.5/0.6 | 2.4/0.5 | 2.6/0.8 | 0.2/0.0 |
| [Evaluation 2] masking/harmonization for urine of cat | 2.3/0.5 | 2.6/0.7 | 2.4/0.8 | 0.4/0.2 |
| [Evaluation 3] masking/harmonization for feces of cat | 2.5/0.6 | 2.6/0.6 | 2.4/0.7 | 0.6/0.1 |

### [Industrial Applicability]

A method for selecting a fragrance excellent in reducing an unpleasant odor has been established by using, as a pseudo malodor, a main substance that causes an unpleasant odor generated from feces and urine of dogs and cats.

Further, the masking fragrance composition prepared by using the deodorant fragrance composition obtained by the selection method described above exhibits an excellent masking effect on an unpleasant odor, and can be suitably used as a pet product, a space deodorant (an air freshener, an air detergent, or the like), a clothing deodorant, a deodorant for excrement, or the like.

## Claims

1. A deodorant fragrance composition for a feces and urine odor of dogs and cats, the deodorant fragrance composition comprising one or more selected from the following component A and one or more selected from the following component B.
Component A: 8-mercaptomenthone, 1-(5,6,7,8-tetrahydro-2-naphthalenyl)ethanone, 2,4,6-trimethyl-4-phenyl-1,3-dioxane, and ethyleneblassylate
Component B: mint oil, benzyl acetate, citronellol, (2-tert-butylcyclohexyl)acetate, (4-tert-butylcyclohexyl)acetate, allylamylglycolate, linalool, α-Ionone, and allylheptanoate

2. The deodorant fragrance composition for a urine odor of a dog according to claim 1, wherein the component A is 1-(5,6,7,8-tetrahydro-2-naphthalenyl)ethanone, and the components B are citronellol and allylheptanoate.

3. The deodorant fragrance composition for a feces and urine odor of a cat according to claim 1, wherein the components A are 8-mercaptomenthone and 2,4,6-trimethyl-4-phenyl-1,3-dioxane, and the components B are mint oil and allylamylglycolate.

4. A method for masking a feces and urine odor of dogs and cats, the method comprising:
using the deodorant fragrance composition according to any one of claims 1 to 3.

5. A pet product comprising:
the deodorant fragrance composition according to any one of claims 1 to 3.

6. Use of a fragrance composition for deodorizing a feces and urine odor of dogs and cats, the fragrance composition comprising one or more selected from the following component A and one or more selected from the following component B.
Component A: 8-mercaptomenthone, 1-(5,6,7,8-tetrahydro-2-naphthalenyl)ethanone, 2,4,6-trimethyl-4-phenyl-1,3-dioxane, and ethyleneblassylate
Component B: mint oil, benzyl acetate, citronellol, (2-tert-butylcyclohexyl)acetate, (4-tert-butylcyclohexyl)acetate, allylamylglycolate, linalool, α-Ionone, and allylheptanoate

7. The use of a fragrance composition for deodorizing a urine odor of a dog according to claim 6, wherein the component A is 1-(5,6,7,8-tetrahydro-2-naphthalenyl)ethanone, and the components B are citronellol and allylheptanoate.

8. The use of a fragrance composition for deodorizing a feces and urine odor of a cat according to claim 6, wherein the components A are 8-mercaptomenthone and 2,4,6-trimethyl-4-phenyl-1,3-dioxane, and the components B are mint oil and allylamylglycolate.
